# DEMANDE DE BREVET EUROPEEN

(11) **EP 0 521 796 A1**
(43) Date de publication de la demande: **07.01.1993**
(21) Numéro de dépôt: 92401918.5
(22) Date de dépôt: 03.07.1992
(51) Int. Cl.: C07C 69/92, C07C 67/31

(54) **Procédé de préparation d'esters d'acides alkoxyphénoxy benzoiques et leur utilisation pour la préparation d'acides (hydroxy-phénoxy) benzoiques**

(30) Priorité: 05.07.1991 FR 9108474
(71) Demandeur: ELF ATOCHEM S.A., F-92800 Puteaux (FR)
(72) Inventeur: Gillet, Jean-Philippe, F-69530 Brignais (FR)
(74) Mandataire: Ahner, Francis

(57) **Abrégé**

La présente invention a pour objet un procédé de préparation d'esters d'acides (alkoxyphénoxy) benzoïques, caractérisé en ce que l'on met en contact un ester d'alkyle ayant au moins trois atomes de carbone d'un acide hydroxybenzoïque avec un dérivé d'halogénoanisole, de préférence en excès, en présence de cuivre ou de l'un de ses dérivés, d'une base et éventuellement d'un agent de transfert de phase,
et en ce que l'on récupère le composé.

Elle a également pour objet les esters nouveaux obtenus et leur utilisation pour préparer les acides (hydroxyphénoxy) benzoïques correspondants.

## Description

La présente invention concerne un procédé de préparation d'ester d'acides (alkoxyphénoxy) benzoïques et l'utilisation de ces derniers pour préparer les acides (hydroxyphénoxy) benzoïques correspondants.

Elle concerne également en tant que produit nouveau, les esters d'acides (alkoxyphénoxy) benzoïques.

Elle concerne enfin un procédé de préparation d'acides (alkoxyphénoxy) benzoïques.

La préparation de l'acide 4-(4'-alkoxyphénoxy)-benzoïque a été décrite par Harington dans Biochem. J., 1926, 20, 300. Cette préparation a ensuite été reprise par J. Walker dans J. Chem. Soc. (1942) p.347-53. Le principe de la réaction est de faire réagir le bromoanisole sur le parahydroxybenzoate d'éthyle en présence de carbonate neutre de potassium (K₂CO₃) et de cuivre. L'intermédiaire est ensuite traité à la potasse et déméthylé en milieu acide acétique et bromhydrique. Cependant, ce procédé requiert une grande quantité de cuivre (14 %) et génère une quantité importante d'effluents. De ce fait, de grandes quantités d'acide acétique et d'acide bromhydrique sont perdues du fait de leur dilution dans l'eau.

Il a également été proposé par Ungnade et col dans J. Org. Chem. 16, 1311-26 (1951) un procédé de préparation voisin, faisant réagir le bromoanisole sur un hydroxybenzoate de potassium, le produit obtenu étant ensuite déméthylé de diverses façons, notamment par la potasse, l'acide iodhydrique ou le trichlorure d'aluminium. Les rendements selon ce procédé sont néanmoins assez faibles.

Plus récemment, le brevet US 3 763 210 a décrit un procédé de préparation à partir de p-méthoxyphénol et de p-fluorobenzoate d'éthyle en présence de soude. Le produit de condensation est isolé, saponifié et déméthylé. Ce procédé nécessite cependant une matière première plus onéreuse et trois étapes.

Les brevets JP 63.099036 et JP 63.104945 décrivent un procédé à partir du 4,4′ diodo diphényléther par monocarbonylation et clivage de l'iodure restant à la soude. Ce procédé s'accompagne d'un recyclage de l'iode. La mise en oeuvre d'un tel procédé semble très délicate à cause de l'iode qui se sublime facilement.

Une autre technique a été décrite dans le brevet EP-A-321 857. Elle implique de partir du 4′-hydroxy 4-phénoxy acétophénone successivement bloqué par acétylation, oxydé à l'air puis débloqué à la soude. Ce procédé de préparation présente l'inconvénient d'une synthèse longue (trois étapes) et de partir d'un composé assez élaboré.

Récemment, les brevets US 4 945 450 et US 4 946 926 ont décrit un procédé de préparation partant du 4-méthoxy phénol et de 4-chloro benzonitrile. Le produit intermédiaire est ensuite traité par les acides acétique et bromhydrique. Dans ce cas, la mise en oeuvre est également difficile du fait des durées de réaction qui sont longues et du fait que le produit final nécessite une purification difficile.

En dernier lieu, Yeager et col dans Synthesis, page 63-68 (1991) ont décrit une voie d'accès à partir du 4-méthoxy phénol que l'on fait réagir sur le 4-fluorobenzaldéhyde en présence de carbonate neutre de potassium dans la DMAC. L'intermédiaire conduit par réaction de Baeyer-Villiger à l'hydroxy ester, ce dernier pouvant donner l'hydroxy acide par saponification.

L'invention a notamment pour but d'améliorer le procédé tel qu'il a été décrit dans Walker ci-dessus mentionné.

Elle propose notamment un procédé offrant de meilleurs rendements de réaction, faisant intervenir deux étapes de réaction seulement, et évitant les traitements laborieux acide/base pour isoler le méthoxy acide.

L'invention concerne en premier lieu un procédé de préparation d'esters d'acides (alkoxyphénoxy) benzoïques de formule : 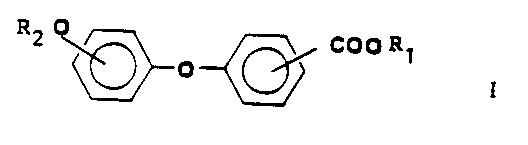
dans laquelle
R₁ est un radical alkyle ayant au moins trois atomes de carbone,
R₂ est un radical alkyle, de préférence (C₁-C₆) alkyle,
caractérisé en ce que l'on met en contact un ester d'un acide hydroxybenzoïque de formule : 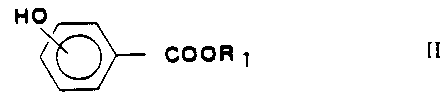
avec un dérivé d'halogénoanisole, de formule : 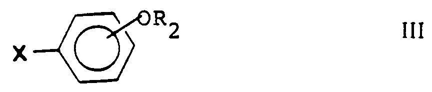
dans laquelle :
X est un atome d'halogène choisi parmi le chlore, le brome, le fluor ou l'iode,
en présence du cuivre ou de l'un de ses dérivés, d'une hase et éventuellement d'un agent de transfert de phase,
et en ce que l'on récupère le composé de formule I.

Dans la présente description, le terme alkyle signifie que les radicaux peuvent être soit linéaires, soit ramifiés. Parmi les radicaux alkyle, on peut citer le méthyle, l'éthyle, le n-propyle, l'isopropyle, le n-butyle, l'isobutyle, le ter-butyle, le n-pentyle.

De préférence, le radical hydroxy de l'ester de formule II est en position méta ou para par rapport au radical COOR₁. De même, on préfèrera les halogénoanisoles de formule III, dans lesquels le radical alkoxy est en position méta ou para par rapport à l'atome d'halogène. La position para pour les deux composés conduit aux résultats les plus avantageux.

De préférence encore, le radical R₁ est choisi parmi les radicaux (C₃-C₆) alkyle, linéaires ou ramifiés, avantageusement R₁ sera choisi parmi le n-propyle ou le n-butyle.

De préférence encore, X est choisi parmi l'atome de brome ou de chlore et/ou OR₂ est le radical méthoxy.

La température de la réaction sera avantageusement comprise entre 140 et 220°C, de préférence entre 180 et 210°C.

La réaction est de préférence effectuée avec un excès d'halogénoanisole de formule III. Généralement, le rapport molaire du composé de formule III par rapport au composé de formule II sera compris entre 1,05 et 2 et il sera de préférence inférieur à 1,4 notamment pour des raisons de mise en oeuvre industrielle.

Les dérivés du cuivre sont choisis parmi le cuivre métallique, les oxydes cuivreux et cuivriques, les halogénures (notamment Cl, Br, I) cuivriques et cuivreux, l'acétate de cuivre. Ces composés peuvent être utilisés seuls ou en mélanges.

De préférence, le rapport molaire du cuivre ou de l'un de ses dérivés par rapport au composé de formule II est compris entre 0,001 et 0,07, avantageusement entre 0,01 et 0,05.

Parmi les bases convenant dans le cadre de présente invention, on peut citer les carbonates de métal alcalin, notamment le carbonate neutre de potassium.

Avantageusement, le rapport molaire de la base par rapport au composé de formule II est compris entre 0,5 et 1,2, de préférence inférieur à 0,75.

Les agents de transfert de phase utilisables dans le cadre de ce procédé sont bien connus de l'homme de métier.

Par agent de transfert de phase, on entend de préférence un composé polyéthoxylé à longue chaîne, du type antarox CO 990 (nonyl phénol oxyéthyléné comportant en moyenne 100 motifs d'oxyde d'éthylène).

Il a été trouvé qu'il était avantageux selon une autre variante de la présente invention de mettre en oeuvre le procédé en présence d'un ligand du cuivre. En effet, cela permet d'améliorer sensiblement le rendement de la condensation par une meilleure conversion et une plus grande sélectivité.

Parmi ces ligands, on peut citer la 8-hydroxyquinoléine, l'acide picolinique, l'acide quinaldique ou l'un de leurs dérivés.

Sans vouloir être limité par une quelconque interprétation scientifique, le déposant pense que ces composés interviennent comme ligand de l'espèce active qui est le Cu^{I}.

L'emploi d'un ligand permet, par ailleurs, de réduire la quantité de catalyseur. De préférence, on emploiera un rapport molaire ligand/composé de formule II compris entre 0,001 et 0,07, préférentiellement entre 0,01 et 0,05.

L'invention a encore pour objet un procédé de préparation du 4-(4′-méthoxy phénoxy) benzoate de propyle ou de butyle, caractérisé en ce que l'on met en contact le 4-hydroxy benzoate de propyle ou de butyle (IIa) avec le 4-bromoanisole (IIIa) dans un rapport molaire IIa/IIIa compris entre 1,05 et 2, de préférence inférieur à 1,4, en présence de carbonate neutre de potassium dans un rapport molaire par rapport à IIa compris entre 0,5 et 1,2, de pérérence inférieur à 0,75 de cuivre métallique ou de l'un de ses dérivés dans un rapport molaire par rapport à IIa compris entre 0,001 et 0,07, de préférence entre 0,01 et 0,05 éventuellement en présence d'un agent de transfert de phase et/ou d'un ligand de cuivre.

Un moyen avantageux de récupérer le produit de formule I consiste en fin de réaction à ajouter un solvant pour faciliter la filtration des sels formés. Parmi les solvants pouvant convenir, on peut citer les solvants polaires, tels que le diméthylformamide, le diméthylsulfoxide.

Le filtrat organique est ensuite distillé pour récupérer d'une part le produit de formule I, et d'autre part, les réactifs n'ayant pas réagi mais qui peuvent être recyclés tels que l'halogénoanisole de formule III qui était présent en excès dans la réaction ou le composé de départ de formule II n'ayant pas réagit.

Parmi les sels formés qui ont été récupérés par filtration comme indiqué ci-dessus, la forme saline du composé de formule I est récupérée par acidification du sel d'acide en milieu aqueux. En effet, lors de la réaction selon l'invention, une partie souvent faible de l'ester de formule I a été. hydrolysée et transformée en sel correspondant (il s'agira du sel de métal alcalin, comme le potassium si l'on a utilisé en tant que base le carbonate neutre de potassium).

Lorsque l'on a utilisé comme base un carbonate neutre de métal alcalin, la solution d'halogènure alcalin issue de ce traitement peut conduire à un recyclage de l'halogène par du chlore. Dans le cas où X est l'atome de brome, le recyclage par Cl₂ est très avantageux.

L'invention a également pour objet les esters d'acide (alkoxy-phénoxy) benzoïque de formule : 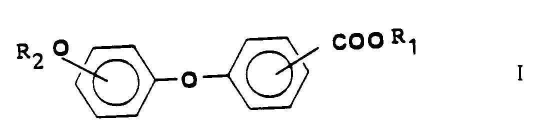
dans laquelle :
R₁ est un radical alkyle ayant au moins trois atomes de carbone,
R₂ est un radical alkyle, de préférence (C₁-C₆) alkyle.

De préférence, les composés sont choisis parmi le 4-(4′-méthoxy phénoxy) benzoate de propyle ou le 4-(4′-méthoxy phénoxy) benzoate de butyle.

Les composés de formule I tels que définis ci-dessus permettent avec une grande facilité et en une seule étape de préparer les acides (hydroxyphénoxy) benzoïques de formule :

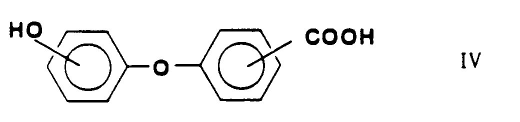

Ces hydroxyacides sont utilisés notamment en tant que monomère dans la synthèse des polyesters thermotropes.

Les composés de formule IV sont obtenus par déblocage simultané, c'est-à-dire hydrolyse, des fonctions éther et ester du composé de formule I.

Il est à noter en conséquence que par rapport à l'art antérieur précédemment discuté, les composés de formule I permettent d'obtenir en une seule étape les acides correspondants de formule IV.

Cette hydrolyse est généralement effectuée en présence d'acide bromhydrique et d'acide acétique à une température généralement comprise entre 80 et 130°C, de préférence entre 100 et 120°C.

Selon une variante avantageuse, les halogénures d'alkyle et les esters correspondants provenant de l'hydrolyse sont distillés en continu à travers une colonne sous forme d'azéotrope avec l'eau.

L'acide de formule IV, qui cristallise en fin de réaction, peut être utilisé tel quel sans qu'il soit nécessaire de le soumettre à une opération de purification.

Selon un mode de mise en oeuvre particulier du procédé selon l'invention, les acides du milieu réactionnel peuvent être recyclés après réajustage de leurs concentrations respectives.

Avantageusement, le rapport molaire de l'acide acétique par rapport à l'ester de formule I est compris entre 3 et 18, de préférence entre 6 et 12.

De préférence, le rapport molaire du composé de formule I par rapport à l'acide bromhydrique est compris entre 1 et 10, et de préférence entre 3 et 7.

D'autres acides permettront dans les mêmes conditions que celles indiquées ci-dessus d'hydrolyser l'ester de formule I pour conduire à l'acide de formule IV. Parmi ces acides, on peut citer les mélanges d'acide bromhydrique avec un autre acide organique choisi parmi l'acide formique et l'acide propionique.

L'invention a encore pour objet un procédé de préparation d'acide hydroxyphénoxy benzoïque de formule IV ci-dessus indiquée, caractérisé en ce que l'on prépare dans une première étape l'ester de formule I ci-dessus décrit, selon le mode opératoire indiqué précédemment, y compris avec les différentes variantes mentionnées et en ce que, dans une seconde étape, on hydrolyse l'ester de formule I comme ceci est indiqué dans la description ci-dessus. De préférence, préalablement à la seconde étape, l'ester de formule I sera isolé dans le but d'obtenir le dérivé de formule IV avec une très bonne pureté. Dans le cas contraire, la réaction peut néanmoins avoir lieu, mais nécessite une étape supplémentaire de purification du produit final de formule IV, par exemple par recristallisation. Cette variante présente en outre l'inconvénient de conduire à un milieu devant être recyclé, qui est chargé d'impuretés.

Le fait de passer par l'ester de formule II limite les réactions parasites, offre une stabilité supérieure et un milieu plus fluide. Il évite également les traitements difficiles acide/base pour isoler l'alkoxy acide.

A la lumière de l'exposé ci-dessus, on comprendra que l'enseignement procuré par la présente invention n'est pas limité à la lecture littérale de la description. D'autres variantes, notamment par l'emploi de composés de départ équivalent pourront être mises en oeuvre sans sortir de la portée de ladite invention.

L'invention sera maintenant décrite à l'aide des exemples suivants :

### Préparation de l'hydroxybenzoate de butyle et de propyle

### Exemple 1

Ces esters sont préparés de façon classique à partir de l'acide 4-hydroxy benzoïque (PHB) et des alcools correspondants par distillation azéotropique catalysée à l'APTS. Les rendements sont pratiquement quantitatifs.

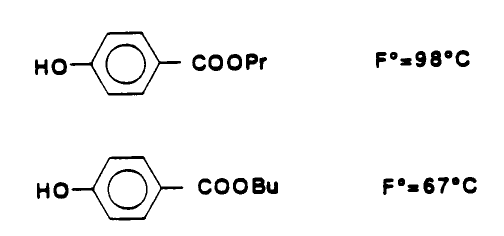

### Préparation du 4-(4′-méthoxy phénoxy) benzoate de propyle

### Exemple 2

Dans un réacteur agité surmonté d'un dean starck, on place 45 g (0,25 M) de 4-hydroxy benzoate de propyle, 52,4 g (0,28M) de 4-bromo anisole, 20,7 g (0,15 M) de carbonate de potassium et 0,8 g de Cu. Le milieu réactionnel est porté à 200°C sous agitation pendant 2 h 30. En fin de réaction, on refroidit le milieu réactionnel et on ajoute 150cm³ de DMF. Les sels formés sont isolés par filtration. Le filtrat contient 0,167 M de 4-(4′-méthoxy phénoxy) benzoate de propyle (dosage CPV). Ce produit est isolé par distillation sous pression réduite. Les sels isolés sont mis en solution aqueuse puis acidifiés. L'acide 4′(4-méthoxy phénoxy) benzoïque qui précipite est isolé par filtration, soit 0,043 mole, de pureté 96,2 % (HPLC). Le rendement global est de 84 % dont 67 % sous la forme ester. L'ester distille à 175°C sous 1 mm Hg.
IR ν (C=0) = 1715 cm⁻¹, ν(C-O) = 1275 cm⁻¹,
ν(C-O-C) = 1232 cm⁻¹.
RMN ¹³C : δ(C=O) 165,7 ppm, δ(CH₃O) = 55,1 ppm, δ(CH₂O) = 65,9 ppm.

### Exemple 3- avec le 8-hydroxyquinoléline

Dans un réacteur agité surmonté d'un dean starck, on place 45 g (0,25 M) de 4-hydroxy benzoate de propyle, 60,7 g (0,325M) de 4-bromo anisole, 20,7 g (0,15 M) de carbonate de potassium et 0,4 g de Cu et 0,9 g de 8-hydroxyquinoléine. Le milieu réactionnel est porté à 198°C pendant 2 h 30. En fin de réaction, on refroidit le milieu et on ajoute 125cm³ de DMF. Les sels formés sont isolés par filtration. Le filtrat contient 0,201 M de 4-(4′-méthoxy phénoxy) benzoate de propyle (dosage CPV). Ce produit est isolé par distillation sous pression réduite. Les sels isolés sont mis en solution aqueuse puis acidifiés. L'acide 4′(4-méthoxy phénoxy) benzoïque qui précipite est isolé par filtration, soit 0,024M de pureté 98,1 % (HPLC). Le rendement global est de 90 % dont 80,4 % sous la forme ester.

### Exemple 4 - identique à l'exemple 3 sans 8-hydroxyquinoéline

Le rendement global est de 82 % dont 69 % sous la forme ester.

### Préparation du 4-(4′-méthoxy phénoxy) benzoate de butyle

### Exemple 5

Dans un montage identique au précédent, on charge 48,5 g (0,25M) de 4-hydroxy benzoate de butyle, 52,4 g (0,28 M) de 4-bromo anisole, 20,7 g (0,15M) de carbonate de potassium et 0,8 g de Cu. Le milieu réactionnel est porté à 198°C pendant 2 h 30 sous agitation. En fin de réaction, on refroidit le milieu réactionnel et on ajoute 175 cm³ de DMF. Les sels formés sont isolés par filtration. Le filtrat contient 0,164M de 4-(4′-méthoxy phénoxy) benzoate de butyle (dosage CPV). Ce produit est isolé par distillation sous pression réduite. Les sels isolés sont mis en solution aqueuse puis acidifiés. L'acide 4-(4′-(méthoxy phénoxy) benzoïque qui précipite est isolé par filtration soit 0,029 mole de pureté 91 % (HPLC). Le rendement global est de 77 % dont 65 % sous la forme ester. L'ester distille à 184°C sous 1 mm Hg.
IR ν(C=O) = 1715 cm⁻¹, ν(C-O) : 1277 cm⁻¹,
ν(C-O-C) = 1232 cm⁻¹,
RMN ¹³C : δ(C=O) 165,8 ppm, δ(CH₃O) : 55,2 ppm, δ(CH₂O) : 64,3 ppm.

### Exemple 6 - avec agent de transfert de phase

On opère comme dans l'exemple 5 mais en ajoutant 5 g d'Antarox 990®. Le rendement global est de 75 % dont 60 % sous la forme ester.

### Préparation du 4-(4′-méthoxy phénoxy) benzoate de méthyle avec agent de transfert de phase (essai comparatif)

### Exemple 7

On opère comme dans l'exemple 6 mais en partant de 4-hydroxy benzoate de méthyle et de CuBr comme catalyseur. Le rendement global est de 49 % dont 35 % sous la forme d'ester.

En opérant dans les conditions générales de l'exemple 5, en présence de Cu et sans agent de transfert de phase, mais en utilisant de l'hydroxybenzoate de méthyle, on obtient les résultats suivants : le rendement global est de 58 % dont 43 % sous la forme d'ester.

### Préparation de l'acide 4-(4′-hydroxy phénoxy) benzoïque

### Exemple 8

Dans un ballon surmonté d'une colonne à distiller, on place 60 g (0,2M) de 4-(4′-méthoxy phénoxy) benzoate de butyle, 120 g (2M) d'acide acétique et 207 g (1,2M) d'acide bromhydrique 47 %. Le milieu réactionnel biphasique est porté à 120°C. Lorsque le régime de la colonne est établi on commence à soutirer en tête les sous-produits de réaction. Le bromure de méthyle est piégé à froid alors que le bromure de butyle, les acétates de méthyle et butyle, l'eau sont récupérés sous la forme d'un biphasique. La réaction dure 2 h 30. Après refroidissement du milieu, l'hydroxy acide précipite. Il est filtré, lavé et séché. Le rendement est de 96,7 %.
F° = 194°-195°C.

### Exemple 9

On utilise la même technique dans l'exemple 8, mais en partant du 4-(4′-méthoxy phénoxy) benzoate de propyle. La réaction est plus rapide 1 h 45. Après refroidissement lavage et filtration recueille l'hydroxy acide pur avec un rendement de 96,4 %.
F° = 194-195°C.

### Exemple 10

Dans un ballon surmonté d'une colonne à distiller, on place 48,8 g (0,2M) d'acide 4-(4′-méthoxy phénoxy) benzoïque obtenu par traitement acide selon les exemples 2 ou 3, 207 g (1,2M) d'acide bromhydrique 47 % et 120 g (2M) d'acide acétique. Le milieu réactionnel est porté à 115°-118°C pendant 2 h 15. Le bromure de méthyle est piégé à froid alors que l'acétate de méthyle est soutiré en tête. Le milieu réactionnel est refroidi. L'hydroxy acide qui précipite est filtré, lavé et séché. On obtient un rendement de 96 %.
F° = 194-195°C.

### Exemple 11

Cet exemple illustre la déméthylation de l'acide 4-(4′-méthoxy phénoxy) benzoïque lorsque l'on opère avec un reflux total.

Dans le même montage que précédemment équipé pour un reflux total, on charge 63,4 g (0,26M) d'acide 4-(4′-méthoxy phénoxy) benzoïque 268,9 g (1,56M) d'HBr 47 % et 110 g (1,83M) d'acide acétique. On porte le milieu à reflux pendant 7 h 30. Puis on refroidit le milieu, on filtre et lave le précipité. On obtient ainsi 55,9 g de produit sec contenant 86,8 % d'acide 4-(4′-hydroxy phénoxy) benzoïque et 8,9 % du produit de départ.

### Exemple 12

Cet exemple illustre la préparation de l'acide 4-(4′-hydroxy phénoxy) benzoïque à partir du 4-(4′-méthoxy phénoxy) benzoate de butyle avec la méthode du reflux total.

Dans un montage équipé pour le reflux total on charge 6 g (0,02M) de 4-(4′-méthoxy phénoxy) benzoate de butyle avec 7,2 g (0,12M) d'acide acétique et 20,7 g (0,12M) d'HBr 47 %. Le milieu réactionnel est porté à reflux pendant 8 h 45. En fin de réaction on refroidit, on filtre et on lave à l'eau. On obtient 4 g de produit sec contenant 73,5 % d'acide 4-(4′ hydroxy phénoxy) benzoïque et 26 % d'acide 4-(4′-méthoxy phénoxy) benzoïque.

On relèvera que dans les exemples mis en oeuvre dans un ballon surmonté d'une colonne à distiller, la cinétique de réaction est nettement améliorée par rapport à une mise en oeuvre utilisant un reflux total. Dans ce dernier cas, d'une part la réaction est beaucoup plus longue, et d'autre part, elle reste souvent inachevée car l'on retrouve dans le milieu réactionnel des reliquats de produit de départ.

Par conséquent, il est avantageux de mettre en oeuvre le procédé selon l'invention en éliminant les sous-produits réactionnels par distillation. Il s'agit principalement des acétates qui se forment dans le milieu réactionnel, notamment l'acétate de méthyle et l'acétate de propyle, ainsi que les bromures correspondants.

On relèvera également que l'acétate éliminé distille sous forme d'un azéotrope, et par suite l'eau non liée à l'acide bromhydrique s'échappe du milieu de réaction, ce qui constitue un avantage supplémentaire lié à ce mode de mise en oeuvre particulier.

## Revendications

1. Procédé de préparation d'esters d'acides (alkoxyphénoxy) benzoïques de formule : 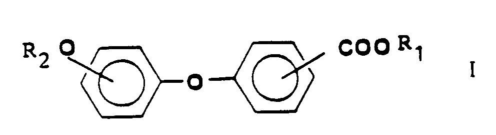 dans laquelle
R₁ est un radical alkyle ayant au moins trois atomes de carbone,
R₂ est un radical alkyle, de préférence (C₁-C₆) alkyle,
caractérisé en ce que l'on met en contact un ester d'un acide hydroxybenzoïque de formule : 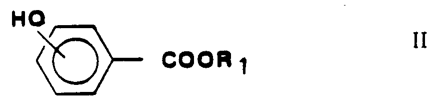 avec un dérivé d'halogénoanisole, de préférence en excès, de formule : 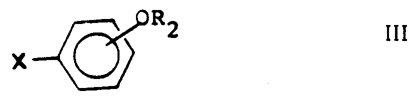 dans laquelle :
X est un atome d'halogène choisi parmi le chlore, le brome, le fluor ou l'iode,
en présence du cuivre ou de l'un de ses dérivés, d'une base et éventuellement d'un agent de transfert de phase,
et en ce que l'on récupère le composé de formule I.

2. Procédé de préparation selon la revendication 1, caractérisé en ce que le radical hydroxy de l'ester de formule II est en position méta ou para par rapport au radical COOR₁.

3. Procédé de préparation selon la revendication 1, caractérisé en ce que le radical alkoxy OR₂ du dérivé d'halogénoanisole de formule III est en position méta ou para par rapport à l'atome d'halogène.

4. Procédé de préparation selon la revendication 1, caractérisé en ce que R₁ est choisi parmi les radicaux (C₁-C₆) alkyle linéaires ou ramifiés.

5. Procédé de préparation selon la revendication 4, caractérisé en ce que R₁ est choisi parmi le n-propyle ou le n-butyle.

6. Procédé de préparation selon la revendication 1 ou 3, caractérisé en ce que X est choisi parmi l'atome de brome ou de chlore et/ou OR₂ est le radical méthoxy.

7. Procédé de préparation selon l'une des revendications précédentes, caractérisé en ce que le rapport molaire du composé III par rapport au composé II est compris entre 1,05 et 2, de préférence inférieur à 1,4.

8. Procédé de préparation selon la revendication 1, caractérisé en ce que les dérivés du cuivre sont choisis parmi le cuivre métallique, les oxydes cuivreux et cuivrique, les halogénures (Cl, Br, I) cuivrique et cuivreux, l'acétate de cuivre, seuls ou en mélange.

9. Procédé de préparation selon la revendication 1 ou 8, caractérisé en ce que le rapport molaire du cuivre ou de l'un de ses dérivés par rapport au composé de formule II est compris entre 0,001 et 0,07 de préférence entre 0,01 et 0,05.

10. Procédé de préparation selon la revendication 1, caractérisé en ce que la base est un carbonate de métal alcalin, notamment le carbonate neutre de potassium.

11. Procédé de préparation selon la revendication 1 ou 10, caractérisé en ce que le rapport molaire de la base par rapport au composé de formule II est compris entre 0,5 et 1,2, de préférence inférieur à 0,75.

12. Procédé de préparation du 4-(4′-méthoxy phénoxy) benzoate de propyle ou de butyle, caractérisé en ce que l'on met en contact le 4-hydroxy benzoate de propyle ou de butyle (IIa) avec le 4-bromoanisole (IIIa) dans un rapport molaire IIa/IIIa compris entre 1,05 et 2, de préférence inférieur à 1,4, en présence de carbonate neutre de potassium dans un rapport molaire par rapport à IIa compris entre 0,5 et 1,2, de préférence inférieur à 0,75, de cuivre métallique ou de l'un de ses dérivés dans un rapport molaire par rapport à IIa compris entre 0,001 et 0,07 de préférence entre 0,01 et 0,05, éventuellement en présence d'un agent de transfert de phase.

13. Procédé selon l'une des revendications précédentes, caractérisé en ce que la réaction est effectuée en présence d'un ligand du cuivre, de préférence, le 8-hydroxyquinoléine.

14. Procédé selon la revendication 13, caractérisé en ce que le rapport molaire du ligand par rapport au composé de formule II est compris entre 0,001 et 0,07, préférentiellement entre 0,01 et 0,05.

15. Procédé selon l'une des revendications 1 à 14, caractérisé en ce que les sous-produits réactionnels sont éliminés du milieu par distillation.

16. Ester d'acide (alkoxyphénoxy) benzoïque de formule : 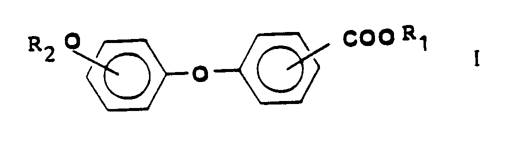 dans laquelle :
R₁ est un radical alkyle ayant au moins trois atomes de carbone,
R₂ est un radical alkyle, de préférence (C₁-C₆) alkyle.

17. 4-(4′-méthoxy phénoxy) benzoate de propyle ou 4-(4′-méthoxy phénoxy) benzoate de butyle.

18. Utilisation des composés selon les revendications 16 ou 17, pour préparer les acides hydroxyphénoxybenzoïques, de formule IV 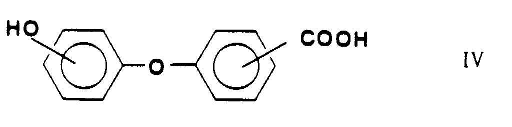 caractérisée en ce que le composé de formule I est hydrolysé.

19. Utilisation selon la revendication 18, caractérisée en ce que l'hydrolyse est effectuée par un mélange acide bromhydrique/acide acétique.
